# FASCICULE DE BREVET EUROPEEN

(11) **EP 2 744 542 B1**
(45) Date de publication et mention de la délivrance du brevet: **18.07.2018**
(21) Numéro de dépôt: 12759796.1
(22) Date de dépôt: 13.08.2012
(51) Int. Cl.: A61M 15/00

(54) **DISPOSITIF D'INHALATION DE POUDRE**
PULVERINHALATOR
POWDER INHALING DEVICE

(30) Priorité: 19.08.2011 FR 1157410
(43) Date de publication de la demande: 25.06.2014
(73) Titulaire: Aptar France SAS, 27110 Le Neubourg (FR)
(72) Inventeur: COLOMB, Arnaud, 78480 Verneuil Sur Seine (FR)
(74) Mandataire: CAPRI
(86) Numéro de dépôt international: PCT/FR2012/051883
(87) Numéro de publication internationale: WO 2013/026976

(56) Documents cités:
- WO-A1-03/035509
- WO-A1-2006/018261
- WO-A1-2011/049541
- WO-A2-2004/067069

## Description

La présente invention concerne un dispositif d'inhalation de poudre, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à emballer les doses de poudre dans des réservoirs individuels prédosés du type blister, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. Ceci rend toutefois les dispositifs complexes et donc coûteux à fabriquer et à assembler. Dans les dispositifs avec bande de blisters, l'assemblage de la bande de blister peut s'avérer complexe, notamment lorsque celle-ci est du type pelable avec un élément de réception rotatif de la couche de base et un élément de réception rotatif de la couche de fermeture. Ainsi, pour assurer un fonctionnement fiable, les couches de base et de fermeture de la bande de blisters devraient être toujours tendues, alors que ceci complique précisément l'assemblage. Les documents WO 2006/018261, WO 03/035509 et WO 2004/067069 décrivent des dispositifs de l'art antérieur. WO2011/049541 A1 divulgue les caractéristiques du préambule de la revendication 1. La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel inhalateur qui soit simple et peu coûteux à fabriquer et à assembler, fiable d'utilisation, avec une bonne précision de dosage et une reproductibilité du dosage à chaque actionnement.

La présente invention a donc pour objet un dispositif d'inhalation de poudre, tel que défini par la revendication 1. Des modes de réalisation avantageux sont définis dans les revendications dépendantes. Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique en section transversale d'un dispositif d'inhalation de poudre selon un mode de réalisation avantageux de l'invention, après assemblage de la bande de blisters et avant mise sous tension des couches de base et de fermeture de ladite bande de blisters,
- la figure 2 est une vue schématique en perspective du dispositif avec les parties de capot ouvertes,
- les figures 3 et 4 sont des vues de détail en section transversale d'une partie du dispositif de la figure 1, respectivement avant et après mise sous tension de la couche de fermeture,
- la figure 5 représente une vue similaire à celle de la figure 1, après assemblage de la bande de blisters et après mise sous tension des couches de base et de fermeture de ladite bande de blisters,
- la figure 6 est une vue schématique en perspective éclatée de l'élément de réception de la couche de fermeture de la bande de blisters,
- les figures 7 et 8 sont des vues en sections transversales, selon des sections différentes, de l'élément de réception de la couche de fermeture de la bande de blisters, en position assemblée,
- la figure 9 est une vue schématique de détail et de dessus d'une partie dudit élément de réception de la couche de fermeture, et
- la figure 10 est une vue schématique de détail et de dessus de l'autre partie dudit élément de réception de la couche de fermeture.

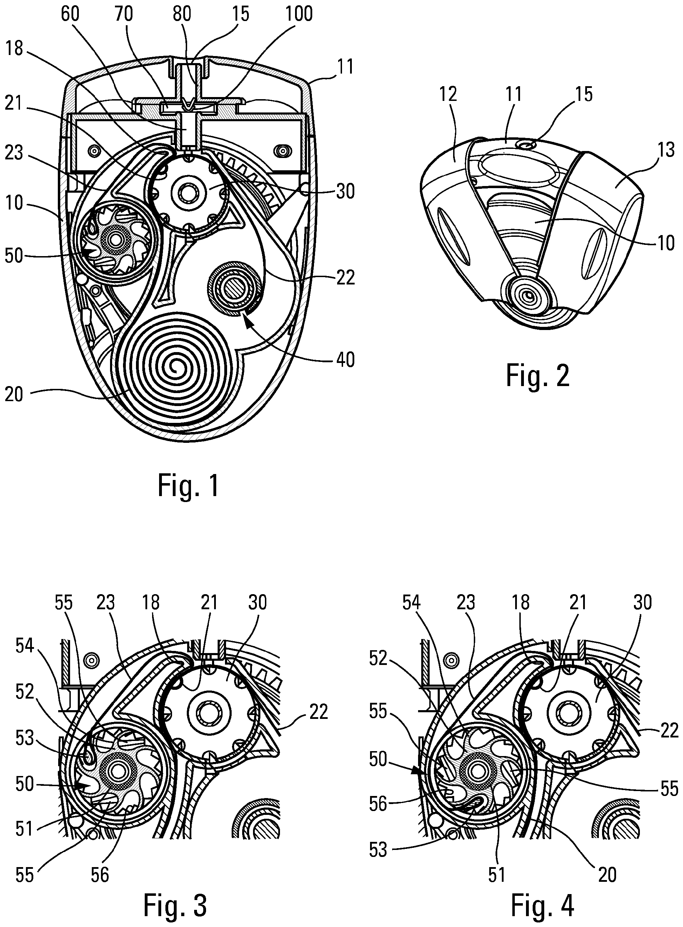

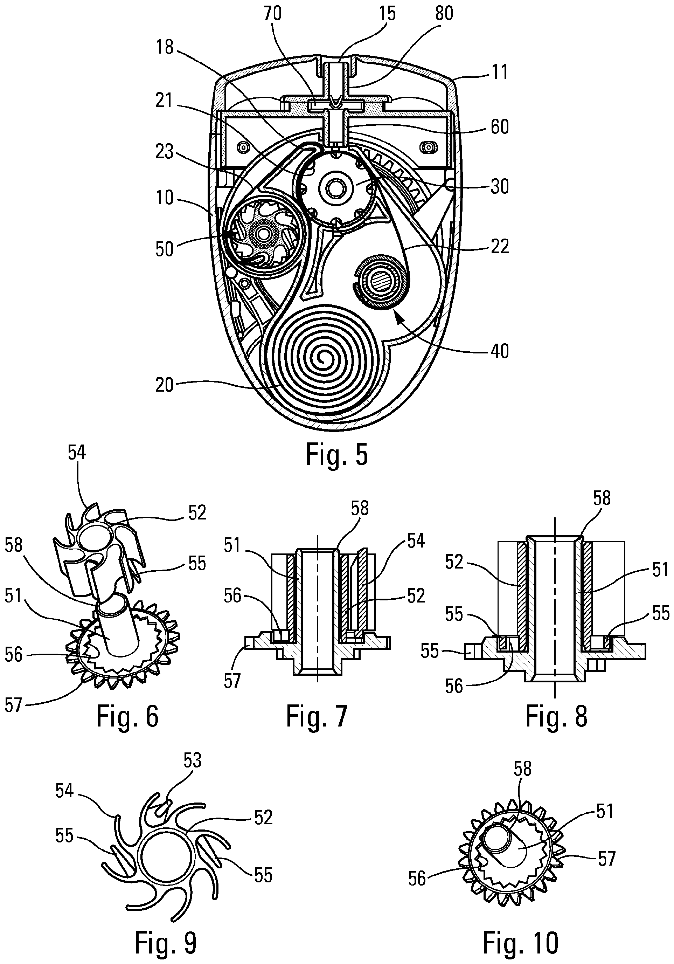

Sur la figure 1 est représentée une variante de réalisation avantageuse d'un inhalateur de poudre sèche. Cet inhalateur comporte un corps 10, sur lequel peuvent être montées coulissantes ou pivotantes deux parties 12, 13 formant capot, visibles sur la figure 6, adaptées à être ouvertes pour ouvrir et charger le dispositif. En variante, une seule partie de capot est aussi possible. Le corps 10 peut être de forme environ arrondie, mais il pourrait avoir toute autre forme appropriée. Le corps 10 comporte un embout buccal 11 définissant un orifice de distribution 15 à travers lequel l'utilisateur va inhaler lors de l'actionnement du dispositif.

A l'intérieur du corps 10, il est prévu une bande 20 de réservoirs individuels prédosés 21, également appelés blisters, réalisée sous la forme d'une bande souple allongée sur laquelle les blisters sont disposés les uns derrière les autres, de manière connue. Cette bande de blisters 20 est du type pelable avec une couche de base 22 comportant les cavités formant les blisters et une couche de fermeture 23 pelable qui se décolle progressivement de la couche de base 22 à chaque actionnement.

Avant la première utilisation, la bande de blister 20 peut être enroulée à l'intérieur du corps 10, de préférence dans une partie de stockage, et des moyens de déplacement de bande 30 sont prévus pour progressivement dérouler et faire avancer cette bande de blister 20. La partie de couche de base 22 comportant les cavités vides est avantageusement adaptée à s'enrouler dans un autre endroit dudit corps 10, notamment une première partie de réception, et la partie de couche de fermeture décollée 23 est avantageusement adaptée à s'enrouler dans encore un autre endroit dudit corps 10, notamment une seconde partie de réception.

La première partie de réception comporte avantageusement un premier élément rotatif de réception 40, autour duquel la partie de couche de base 22 comportant les cavités vides s'enroule. Avantageusement, ce premier élément de réception rotatif 40 est sollicité en rotation par un ressort (non représenté), notamment un ressort à spirale disposé à l'intérieur dudit premier élément de rotation. Ceci garantit un bon enroulement à chaque actionnement.

La seconde partie de réception comporte avantageusement un second élément rotatif de réception 50, autour duquel la partie de couche de fermeture décollée 23 s'enroule. Ce second élément de réception rotatif 50 est également sollicité en rotation, notamment en étant corrélé en rotation avec les moyens de déplacement 30 de la bande de blisters 20. Ceci garantit un bon enroulement à chaque actionnement.

Les moyens de déplacement 30 sont adaptés à faire avancer la bande de blisters 20 avant et/ou pendant chaque actionnement du dispositif. De préférence, les moyens de déplacement comportent une roue d'indexage 30 qui reçoit et guide les blisters. Une rotation de cette roue d'indexage 30 fait avancer la bande de blister 20. Avantageusement, cette roue d'indexage 30 est tournée lorsque le dispositif est chargé, par exemple par l'ouverture du capot.

Selon un aspect avantageux, l'inhalateur comporte une chambre de dispersion 70 qui est destinée à recevoir la dose de poudre après ouverture d'un réservoir respectif. Cette chambre de dispersion 70 comporte une entrée, directement reliée au réservoir ouvert par un canal d'amenée de poudre 60, et une sortie, directement reliée à l'orifice de distribution 15 par un canal de distribution de poudre 80. La chambre de dispersion 70 contient de préférence au moins une bille 71, avantageusement plusieurs billes, par exemple trois. Ces billes 71 peuvent se déplacer dans ladite chambre de dispersion 70 le long d'un chemin de bille sensiblement circulaire qui s'étend dans un plan environ transversal à l'orientation des canaux d'amenée 60 et de distribution 80. La chambre de dispersion 70 comporte au moins une, de préférences deux entrées d'air tangentielles. De cette manière, les flux d'air entrant par ces entrées d'air tangentielles vont tourbillonner dans la chambre de dispersion 70 et entrainer les billes 71 en rotation. Ces flux d'air, qui sont crées par l'inhalation de l'utilisateur vont se mélanger au flux d'air et de poudre qui vient depuis le réservoir ouvert à travers le canal d'amenée 60, et qui pénètre dans la chambre de dispersion 70. Ce flux d'air et de poudre va alors être également entraîné en rotation dans la chambre de dispersion 70, et les billes 71 vont désagglomérer la poudre avant que celle-ci ne sorte de la chambre de dispersion 70 vers l'orifice de distribution 15 via ledit canal de distribution 80. Eventuellement, un déflecteur 100 peut être prévu à l'endroit où débouche le canal d'amenée de poudre 60 dans la chambre de dispersion 70, notamment lorsque ledit canal d'amenée 60 est coaxial et aligné avec le canal de distribution 80, comme représenté sur la figure 1. Ceci permet de dévier le flux d'air et de poudre venant du réservoir ouvert et d'éviter que celui-ci ne s'écoule directement dans le canal de distribution 80. Bien entendu, d'autres forme et positions des canaux d'amenée et de distribution sont possibles.

Lorsque le dispositif est actionné, la roue d'indexage 30 est tournée, ce qui amène le prochain blister 21 face au canal d'amenée 60. Simultanément, la couche de couverture 23 est retirée de ce blister 21, en étant pelée par la rotation du second élément de réception rotatif 50 qui est corrélée à la rotation de la roue d'indexage 30. Avantageusement, la couche de fermeture s'étend autour d'un coin de pelage 18 fixe qui, associé à la rotation simultanée de la roue d'indexage 30 et du second élément de réception rotatif 50 va provoquer le décollement ou pelage de la couche de fermeture 23 de la couche de base 22 au niveau dudit coin de pelage 18. La couche de base 22 en aval de la roue d'indexage 30 va s'enrouler autour du premier élément de réception rotatif 40. Ainsi, un blister ouvert 21 se retrouve face au canal d'amenée 60. Lorsque l'utilisateur inhale, il va créer un premier flux d'air à travers un canal d'arrivée d'air, qui va traverser le blister ouvert et ainsi entraîner la poudre sous la forme d'un flux d'air et de poudre dans le canal d'amenée 60. Simultanément, l'inhalation va créer des flux d'air secondaires qui vont pénétrer tangentiellement dans la chambre de dispersion 70 à travers les entrées d'air tangentielles. Ces flux d'air secondaires vont faire tourner les billes 71 dans la chambre de dispersion 70. Lorsque le flux d'air et de poudre rencontre ces flux d'air secondaires et les billes en mouvement dans la chambre de dispersion 70, il va se créer des tourbillonnements, ce qui va fluidifier et désagglomérer la poudre, qui finalement va s'échapper en direction de l'orifice de distribution 15..

Les dimensions et orientations des différents canaux peuvent bien entendu être optimisées selon les spécificités de l'inhalateur, pour maximiser les performances de celui-ci.

Pour garantir un fonctionnement fiable du dispositif, la couche de base 22 et la couche de fermeture 23, qui s'enroulent respectivement autour des premier et second éléments de réception rotatifs 40, 50, doivent être tendus. Lors de l'assemblage de la bande de blisters dans le corps 10, il est toutefois souhaitable de ne pas avoir de tension sur ces parties de la bande de blisters. Ainsi, comme visible sur la figure 1, qui montre la bande de blisters après son assemblage, aussi bien la couche de fermeture 23 que la couche de base sont détendus, ce qui facilite cet assemblage.

Au niveau de la couche de base 22 et du premier élément de réception 40, celui-ci peut comporter un ressort à spirale qui assure une traction sur ladite couche de base après son assemblage.

Concernant la couche de fermeture 23, celle-ci est fixée à un second élément de réception 50 formée en deux parties. Une première partie 51 appelée roue de pelage, et une seconde partie 52 appelée tendeur, qui est assemblée sur ladite première partie. Dans la suite de la description, ces deux parties seront désignées par les termes roue de pelage 51 et tendeur 52.

La roue de pelage 51 comporte un manchon central comportant d'un coté un plateau pourvu d'une denture externe 57 engrenée avec une denture correspondante (non représentée) de la roue d'indexage 30. Ainsi, chaque rotation de la roue d'indexage entraîne une rotation de la roue de pelage 51 dans une première direction de rotation. Le plateau de la roue de pelage comporte également une denture interne 56 dont l'utilité sera décrite ci-après. De l'autre coté du manchon, la roue de pelage comporte un profil d'encliquetage 58.

Le tendeur 52 comporte un manchon central creux qui s'assemble autour du manchon central de la roue de pelage, et dont le bord supérieur vient s'encliqueter sous le profile d'encliquetage 58 de la roue de pelage, comme visible sur les figures 7 et 8. Du coté opposé, le tendeur comporte deux doigts flexibles 55, de préférences diamétralement opposés, et qui coopère avec la denture interne 56 de la roue de pelage pour former un cliquet. Le tendeur 52 peut donc tourner autour de la roue de pelage 51, mais seulement dans ladite première direction de rotation. Le tendeur comporte aussi un élément de fixation 53 de l'extrémité avant de la couche de fermeture 23 de la bande de blisters, ainsi que des pales souples 54 capables de se déformer radialement vers l'intérieur au fur et à mesure de l'enroulement de ladite couche de fermeture 23. Cette déformation progressive des pales souples 54 permet de maintenir la tension de la couche de fermeture alors même que le diamètre extérieur d'enroulement augmente alors que le pas de rotation de la roue de pelage 51 reste constant.

Lors de l'assemblage, la couche de fermeture n'est pas tendue pour faciliter l'assemblage. La roue de pelage 51 peut alors être bloquée en rotation, en bloquant directement ladite roue de pelage 51 ou en bloquant la roue d'indexage 30, et le tendeur 52 peut alors être tourné par rapport à la roue de pelage 51 dans ladite première direction de rotation pour tendre ladite couche de fermeture 23. Cette opération de tension est illustrée sur les figures 3 et 4. Par la suite, chaque rotation de la roue de pelage 51 dans la première direction de rotation entrainera une rotation correspondante du tendeur 52 et ainsi un enroulement progressif de la couche de fermeture 23 autour dudit second élément de réception rotatif 50.

Diverses modifications sont possibles pour un homme du métier sans sortir du cadre de la présente invention tel que défini par les revendications annexées.

## Revendications

1. Dispositif d'inhalation de poudre, comportant un corps (10) pourvu d'un orifice de distribution (15), une pluralité de réservoirs prédosés (21) contenant chacun une dose de poudre à distribuer, ladite pluralité de réservoirs (21) étant formée sur une bande de blisters (20) souple allongée, comportant une couche de base (22) contenant les cavités des réservoirs et une couche de fermeture (23) recouvrant lesdites cavités, la couche de fermeture (23) étant pelable de la couche de base (22), la partie de couche de base (22) contenant les blisters vides s'enroulant autour d'un premier élément de réception rotatif (40) et la partie de couche de fermeture (23) pelée de ladite couche de base (22) s'enroulant autour d'un second élément de réception rotatif (50), ledit second élément de réception rotatif (50) étant constitué de deux parties, une première partie formant roue de pelage (51) et une seconde partie formant tendeur (52), ledit tendeur (52) pouvant tourner par rapport à ladite roue de pelage (51) seulement dans une première direction de rotation, ledit tendeur (52) tournant ensemble avec ladite roue de pelage (51) dans ladite première direction de rotation pour enrouler ladite couche de fermeture (23) à chaque actionnement du dispositif, **caractérisé en ce que** ladite roue de pelage (51) comporte un manchon central comportant d'un côté un plateau pourvu d'une denture externe (57) et d'une denture interne (56), et comportant de l'autre côté un profil d'encliquetage (58), ledit tendeur (52) comportant un manchon central creux assemblé autour dudit manchon central de ladite roue de pelage (51), le bord supérieur dudit manchon central creux dudit tendeur (52) étant encliqueté sous ledit profile d'encliquetage (58) de ladite roue de pelage (51), ledit tendeur (52) comportant un élément de fixation (53) recevant l'extrémité avant de ladite couche de fermeture (23), et ledit tendeur (52) pouvant tourner par rapport à ladite roue de pelage (51) dans cette première direction de rotation pour tendre ladite couche de fermeture (23) après assemblage de la bande de blisters (20) dans le corps (10).

2. Dispositif selon la revendication 1, dans lequel ladite denture interne (56) coopère avec des doigts flexibles (55) du tendeur formant cliquet et permettant une rotation du tendeur (52) par rapport à ladite roue de pelage (51) dans ladite première direction de rotation et empêchant une rotation en sens inverse.

3. Dispositif selon l'une quelconque des revendications précédentes, dans lequel le dispositif comporte des moyens de déplacement (30), tel qu'une roue d'indexage, pour faire avancer la bande de blisters (20) avant et/ou pendant chaque actionnement, ledit second élément de réception rotatif (50) étant corrélée en rotation avec ladite roue d'indexage (30).

4. Dispositif selon la revendication 3, dans lequel ladite denture externe (57) est engrenée en rotation avec ladite roue d'indexage (30).

5. Dispositif selon l'une quelconque des revendications précédentes, dans lequel ledit tendeur comporte des pales souples (54) adaptées à se comprimer radialement au fur et à mesure de l'enroulement de la couche de fermeture (23).

6. Dispositif selon l'une quelconque des revendications précédentes, dans lequel l'extrémité avant de la couche de base (22) est fixée audit premier élément de réception rotatif (40) qui est pourvu d'un ressort à spirale pour exercer une tension sur ladite couche de base (22).

7. Dispositif selon l'une quelconque des revendications précédentes, dans lequel le dispositif comporte une chambre de dispersion (70) comportant une entrée reliée lors de l'inhalation à un réservoir ouvert (21) et recevant le flux d'air et de poudre à partir dudit réservoir ouvert via un canal d'amenée (60), et une sortie reliée audit orifice de distribution (15) via un canal de distribution (80), ladite chambre de dispersion (70) comportant au moins une bille (71) déplaçable dans ladite chambre de dispersion, ladite chambre de dispersion (70) comportant au moins une entrée d'air environ tangentielle, lesdits canaux d'amenée (60) et de distribution (80) s'étendant dans une même direction sensiblement perpendiculaire à ladite au moins une entré d'air tangentielle (72) de ladite chambre de dispersion (70).

8. Dispositif selon l'une quelconque des revendications précédentes, dans lequel ledit dispositif comporte des moyens pour décoller ladite couche de fermeture (23) de ladite couche de base (22), tels qu'un bord de pelage (18) autour duquel s'étend ladite couche de fermeture (23).

9. Dispositif selon l'une quelconque des revendications précédentes, dans lequel ladite chambre de dispersion (70) contient une pluralité de billes (71).

## Patentansprüche

1. Vorrichtung zur Inhalation eines Pulvers, aufweisend einen mit einer Ausgabeöffnung (15) versehenen Körper (10), mehrere vordosierte Behälter (21), die jeweils eine Dosis von auszugebendem Pulver enthalten, wobei die mehreren Behälter (21) auf einem flexiblen, langgestreckten Blisterstreifen (20) gebildet sind, der eine Grundschicht (22), die die Hohlräume der Behälter enthält, und eine Verschlussschicht (23) aufweist, die die Hohlräume bedeckt, wobei die Verschlussschicht (23) von der Grundschicht (22) abziehbar ist, wobei sich der Teil der Grundschicht (22), der die leeren Blister aufweist, um ein erstes drehbares Aufnahmeelement (40) aufwickelt, und sich der Teil der Verschlussschicht (23), der von der Grundschicht (22) abgezogen wird, um ein zweites drehbares Aufnahmeelement (50) wickelt, wobei das zweite drehbare Aufnahmeelement (50) aus zwei Teilen besteht, wobei ein erster Teil ein Abziehrad (51) und ein zweiter Teil ein Spannstück (52) bildet, wobei sich das Spannstück (52) in Bezug auf das Abziehrad (51) nur in eine erste Drehrichtung drehen kann, wobei sich das Spannstück (52) zusammen mit dem Abziehrad (51) in die erste Drehrichtung dreht, um bei jeder Betätigung der Vorrichtung die Verschlussschicht (23) aufzuwickeln, **dadurch gekennzeichnet, dass** das Abziehrad (51) eine mittige Muffe aufweist, die auf einer Seite eine mit einer Außenverzahnung (57) und einer Innenverzahnung (56) versehene Scheibe aufweist und auf der anderen Seite ein Rastprofil (58) aufweist, wobei das Spannstück (52) eine mittige Hohlmuffe aufweist, die um die mittige Muffe des Abziehrads (51) montiert ist, wobei der obere Rand der mittigen Hohlmuffe des Spannstücks (52) unter dem Rastprofil (58) des Abziehrads (51) eingerastet ist, wobei das Spannstück (52) ein Befestigungselement (53) aufweist, das das vordere Ende der Verschlussschicht (23) aufnimmt, und wobei sich das Spannstück (52) in Bezug auf das Abziehrad (51) in diese erste Drehrichtung drehen kann, um die Verschlussschicht (23) nach der Montage des Blisterstreifens (20) in dem Körper (10) zu spannen.

2. Vorrichtung nach Anspruch 1, wobei die Innenverzahnung (56) mit flexiblen Stiften (55) des Spannstücks zusammenwirkt zum Bilden einer Sperrklinke und Ermöglichen einer Drehung des Spannstücks (52) in Bezug auf das Abziehrad (51) in der ersten Drehrichtung und Verhindern einer Drehung in umgekehrter Richtung.

3. Vorrichtung nach einem der vorhergehenden Ansprüche, wobei die Vorrichtung Verschiebemittel (30), wie ein Schaltrad, aufweist, um den Blisterstreifen (20) vor und/oder während jeder Betätigung vorzuschieben, wobei das zweite drehbare Aufnahmeelement (50) mit dem Schaltrad (30) drehend in Wechselbeziehung steht.

4. Vorrichtung nach Anspruch 3, wobei die Außenverzahnung (57) drehend mit dem Schaltrad (30) im Eingriff ist.

5. Vorrichtung nach einem der vorhergehenden Ansprüche, wobei das Spannstück flexible Blätter (54) aufweist, die dazu geeignet sind, sich im Laufe des Aufwickelns der Verschlussschicht (23) radial zusammenzudrücken.

6. Vorrichtung nach einem der vorhergehenden Ansprüche, wobei das vordere Ende der Grundschicht (22) an dem ersten drehbaren Aufnahmeelement (40) befestigt ist, welches mit einer Spiralfeder versehen ist, um eine Spannung auf die Grundschicht (22) auszuüben.

7. Vorrichtung nach einem der vorhergehenden Ansprüche, wobei die Vorrichtung eine Dispersionskammer (70) aufweist, die einen Einlass, der bei der Inhalation mit einem offenen Behälter (21) verbunden ist und den Strom von Luft und Pulver aus dem offenen Behälter über einen Zufuhrkanal (60) empfängt, und einen Auslass aufweist, der über einen Ausgabekanal (80) mit der Ausgabeöffnung (15) verbunden ist, wobei die Dispersionskammer (70) mindestens eine Kugel (71) aufweist, die in der Dispersionskammer verschiebbar ist, wobei die Dispersionskammer (70) mindestens einen, in etwa tangentialen Lufteinlass aufweist, wobei sich der Zufuhr- (60) und der Ausgabekanal (80) in eine gleiche Richtung im Wesentlichen senkrecht zu dem mindestens einen tangentialen Lufteinlass (72) der Dispersionskammer (70) erstreckt.

8. Vorrichtung nach einem der vorhergehenden Ansprüche, wobei die Vorrichtung Mittel zum Lösen der Verschlussschicht (23) von der Grundschicht (22) aufweist, wie eine Abziehkante (18), um die sich die Verschlussschicht (23) erstreckt.

9. Vorrichtung nach einem der vorhergehenden Ansprüche, wobei die Dispersionskammer (70) mehrere Kugeln (71) enthält.

## Claims

1. A powder inhaler device comprising a body (10) provided with a dispenser orifice (15), a plurality of predosed reservoirs (21), each containing a dose of powder to be dispensed, said plurality of reservoirs (21) being formed on an elongate flexible blister strip (20) comprising a base layer (22) that contains the cavities of the reservoirs, and a closure layer (23) overlying said cavities, the closure layer (23) being peelable off the base layer (22), the portion of base layer (22) containing the empty blisters rolling up around a first rotary receiver element (40), and the portion of closure layer (23) peeled off said base layer (22) rolling up around a second rotary receiver element (50), said second rotary receiver element (50) being constituted by two portions, a first portion forming a peeling wheel (51), and a second portion forming a tensioner (52), said tensioner (52) being capable of turning relative to said peeling wheel (51) in a first direction of rotation only, said tensioner (52) turning together with said peeling wheel (51) in said first direction of rotation so as to roll up said closure layer (23) at each actuation of the device, **characterized in that** said peeling wheel (51) comprises a central sleeve including on one end, a disk that is provided with an outer set of teeth (57) and an inner set of teeth (56), and including on the other end, a snap-fastener profile (58), said tensioner (52) including a hollow central sleeve that is assembled around said central sleeve of said peeling wheel (51), the top edge of said hollow central sleeve of said tensioner (52) being snap-fastened below said snap-fastener profile (58) of said peeling wheel (51), said tensioner (52) including a fastener element (53) that receives the leading end of said closure layer (23), and said tensioner (52) being capable of turning relative to said peeling wheel (51) in the first direction of rotation so as to tension said closure layer (23) after the blister strip (20) has been assembled in the body (10).

2. A device according to claim 1, wherein said inner set of teeth (56) cooperates with pawl-forming flexible fingers (55) of the tensioner, which pawls enable the tensioner (52) to turn relative to said peeling wheel (51) in said first direction of rotation, but prevent it from turning in the opposite direction.

3. A device according to either preceding claim, wherein the device includes displacement means (30), such as an indexer wheel, so as to cause the blister strip (20) to advance before and/or during each actuation, said second rotary receiver element (50) being correlated in rotation with said indexer wheel (30).

4. A device according to claim 3, wherein said outer set of teeth (57) meshes in rotary manner with said indexer wheel (30).

5. A device according to any preceding claim, wherein said tensioner includes flexible blades (54) that are adapted to compress radially as the closure layer (23) rolls up.

6. A device according to any preceding claim, wherein the leading end of the base layer (22) is fastened to said first rotary receiver element (40) that is provided with a spiral spring so as to exert tension on said base layer (22).

7. A device according to any preceding claim, wherein the device includes a dispersion chamber (70) including both an inlet that, during inhalation, is connected to an open reservoir (21), and receives the flow of air and of powder from said open reservoir via a delivery channel (60), and also an outlet that is connected to said dispenser orifice (15) via a dispenser channel (80), said dispersion chamber (70) including at least one ball (71) that is movable in said dispersion chamber, said dispersion chamber (70) including at least one approximately tangential air inlet, said delivery and dispenser channel (60 and 80) extending in a same direction that is substantially perpendicular to said at least one tangential air inlet (72) of said dispersion chamber (70).

8. A device according to any preceding claim, wherein said device includes means for unsticking said closure layer (23) from said base layer (22), such as a peeling edge (18) around which said closure layer (23) extends.

9. A device according to any preceding claim, wherein said dispersion chamber (70) contains a plurality of balls (71).
